# EUROPÄISCHE PATENTANMELDUNG

(11) **EP 1 894 922 A1**
(43) Veröffentlichungstag der Anmeldung: **05.03.2008**
(21) Anmeldenummer: 06012792.5
(22) Anmeldetag: 22.06.2006
(51) Int. Cl.: C07D 317/38

(54) **Verfahren zur Herstellung von Glycerincarbonat-Estern (II)**

(71) Anmelder: Cognis GmbH, 40589 Düsseldorf (DE)
(72) Erfinder: Westfechtel, Alfred, Dr., 40724 Hilden (DE)
(74) Vertreter: Herzog, Martin

(57) **Zusammenfassung**

Vorgeschlagen wird ein Verfahren zur Herstellung von Glycerincarbonat-Estern der Formel (I) 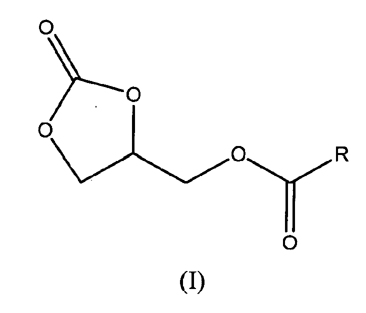 worin R Wasserstoff oder einen Alkyl- oder Alkenylrest mit 1 bis 23 C-Atomen bedeutet, der geradkettig oder verzweigt sein kann, wobei man in Gegenwart eines Umesterungs-Katalysators eine Verbindung der Formel (II) 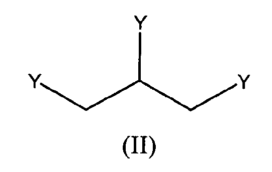 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat und einem Alkylester der Formel (IV) 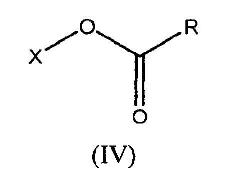 worin R die oben genannte Bedeutung hat und X einen Alkyl- oder Alkenylrest mit 1 bis 8 C-Atomen bedeutet, der linear oder verzweigt sein kann, umestert, mit der Maßgabe, dass die Gruppe R der Verbindung (IV) verschieden von der Gruppe Z der Verbindung (II) ist.

## Beschreibung

### Gebiet der Erfindung

Die Erfindung betrifft ein Verfahren zur Herstellung von Glycerincarbonat-Estern.

### Stand der Technik

Ester des Glycerincarbonats sind relativ wenig beschrieben. Gemäß Zephirin Mouloungui et al., Eur. J. Lipid Sci. Technol., 103 (2001) 216 - 222 werden die langkettigen Carbonat-Ester durch Umsetzung von Glycerin-Carbonat mit Säurechloriden erhalten. Im Rahmen einer Übersicht zu cyclischen Carbonat-funktionellen Polymeren stellt Dean C. Webster in Progress in Organic Coatings, 47 (2003), 77-86 mehrere Synthesewege von Glycerincarbonatmethacrylat vor, unter anderem die Umesterung von Glycerincarbonat mit Methylmethacrylat. US 3,225,063 beschreibt die Darstellung von Carbonat-Estern mittels cyclischer Anhydride in Form saurer Halbester.

Acrylaten und Methacrylaten als ungesättigten Estern des Glycerincarbonats sind spezielle Publikationen gewidmet. So beschreibt US 2,979,514 die Umsetzung von Säurechloriden und Glycerincarbonat. Dabei wird mit großen Überschüssen an Reagenzien und Lösemitteln bzw. Schleppmitteln gearbeitet. In der DE 10355830 A1 wird ein Verfahren vorgeschlagen, bei dem Metall-Chelat-Katalysatoren für die Umesterung von Glycerincarbonat mit Methylmethacrylat eingesetzt werden. Auch hierbei wird ein hoher Überschuss Acrylat verwandt.

WO 93/09111 A2 offenbart ein Verfahren zur Herstellung von Glycerincarbonat-Estem, wobei Glycerinester mit C₁₋₂₃ Fettsäuren, insbesondere Triglyceride auf Basis der genannten Fettsäuren, in Gegenwart eines Katalysators mit einem Carbonat, insbesondere Dimethylcarbonat oder Diethylcarbonat umgesetzt werden.

### Beschreibung der Erfindung

Aufgabe der vorliegenden Erfindung war es, ein neues Verfahren zur Herstellung von Glycerincarbonat-Estern bereitzustellen.

Die Erfindung betrifft ein Verfahren zur Herstellung von Glycerincarbonat-Estern der Formel (I) 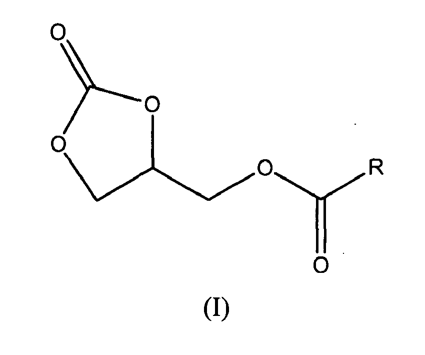 worin R Wasserstoff oder einen Alkyl- oder Alkenylrest mit 1 bis 23 C-Atomen bedeutet, der geradkettig oder verzweigt sein kann, wobei man in Gegenwart eines Umesterungs-Katalysators eine Verbindung der Formel (II) 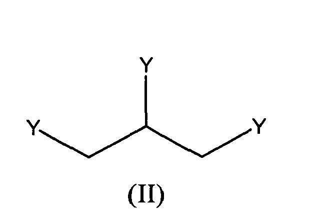 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat und einem Alkylester der Formel (IV) 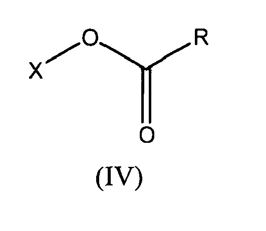 worin R die oben genannte Bedeutung hat und X einen Alkyl- oder Alkenylrest mit 1 bis 8 C-Atomen bedeutet, der linear oder verzweigt sein kann, umestert, mit der Maßgabe, dass die Gruppe R der Verbindung (IV) verschieden von der Gruppe Z der Verbindung (II) ist.

Ausdrücklich sei klargestellt, dass sich das Wort "oder" in dem Ausdruck "Dimethylcarbonat oder Diethylcarbonat" nur auf die beiden Carbonate bezieht, Es werden also beim erfindungsgemäßen Verfahren drei Reaktionskomponenten miteinander umgesetzt und zwar
• Verbindung (II),
• Dimethylcarbonat oder Diethylcarbonat,
• Verbindung (IV)

In einer Ausführungsform wird das erfindungsgemäße Verfahren in zwei Schritten durchgeführt. In dieser Ausführungsform betrifft die Erfindung ein Verfahren zur Herstellung von Glycerincarbonat-Estern der Formel (I) 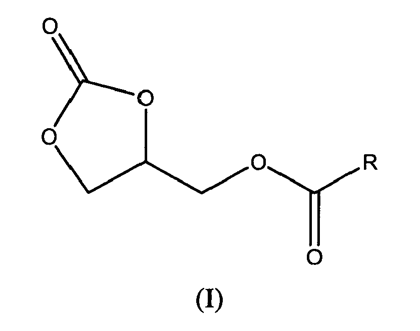 worin R Wasserstoff oder einen Alkyl- oder Alkenylrest mit 1 bis 23 C-Atomen bedeutet, der geradkettig oder verzweigt sein kann, wobei man in einem ersten Schritt in Gegenwart eines Umesterungs-Katalysators eine Verbindung der Formel (II) 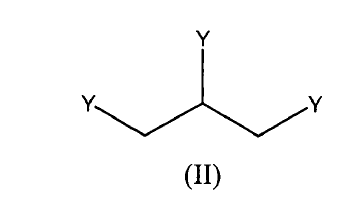 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat zu einer Verbindung der Formel (III) 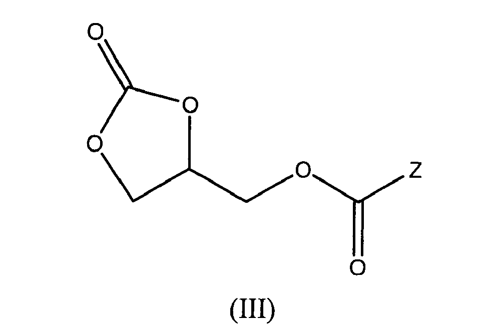 worin Z die oben genannte Bedeutung hat, umsetzt und die Verbindung (III) in einem zweiten Schritt in Gegenwart eines Umesterungs-Katalysators mit einem Alkylester der Formel (IV) 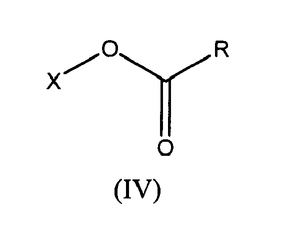 worin R die oben genannte Bedeutung hat und X einen Alkyl- oder Alkenylrest mit 1 bis 8 C-Atomen bedeutet, der linear oder verzweigt sein kann, umestert, mit der Maßgabe, dass die Gruppe R der Verbindung (IV) verschieden von der Gruppe Z der Verbindung (II) ist.

Obgleich für den Fachmann ohne weiteres verständlich, sei ausdrücklich der Sinn der Maßgabe, dass "die Gruppe R der Verbindung (IV) verschieden von der Gruppe Z der Verbindung (II) ist", klargestellt. Diese Maßgabe bedeutet:
• Falls die Zielverbindung (I) mit R = Wasserstoff hergestellt werden soll, dann müssen solche Verbindungen (IV) eingesetzt werden, bei denen R Wasserstoff ist; mithin darf die Gruppe Z der Verbindungen (II) nicht Wasserstoff sein.
• Falls die Zielverbindung (I) mit R = Methyl hergestellt werden soll, dann müssen solche Verbindungen (IV) eingesetzt werden, bei denen R Methyl ist; mithin darf die Gruppe Z der Verbindungen (II) nicht Methyl sein.
• Falls die Zielverbindung (I) mit R = Ethyl hergestellt werden soll, dann müssen solche Verbindungen (IV) eingesetzt werden, bei denen R Ethyl ist; mithin darf die Gruppe Z der Verbindungen (II) nicht Ethyl sein.
• Falls die Zielverbindung (I) mit R = n-Propyl hergestellt werden soll, dann müssen solche Verbindungen (IV) eingesetzt werden, bei denen R n-Propyl ist; mithin darf die Gruppe Z der Verbindungen (II) nicht n-Propyl sein.
• Falls die Zielverbindung (I) mit R = i-Propyl hergestellt werden soll, dann müssen solche Verbindungen (IV) eingesetzt werden, bei denen R i-Propyl ist; mithin darf die Gruppe Z der Verbindungen (II) nicht i-Propyl sein.
• Falls die Zielverbindung (I) mit R = -CH=CH₂ hergestellt werden soll, dann müssen solche Verbindungen (IV) eingesetzt werden, bei denen R -CH=CH₂ ist; mithin darf die Gruppe Z der Verbindungen (II) nicht -CH=CH₂ sein.
• Falls die Zielverbindung (I) mit R = -C(CH₃)=CH₂ hergestellt werden soll, dann müssen solche Verbindungen (IV) eingesetzt werden, bei denen R -C(CH₃)=CH₂ ist; mithin darf die Gruppe Z der Verbindungen (II) nicht -C(CH₃)=CH₂ sein.

Der Grund für die Maßgabe wird ohne weiteres offensichtlich, wenn man die oben genannten 2-stufige Durchführung des erfindungsgemäßen Verfahrens betrachtet. Hierbei wird in Schritt 1 zunächst die Zwischenverbindung (III) erhalten, bei der in Schritt 2 formal der Rest Z gegen den Rest R ausgetauscht wir, wobei die Zielverbindung (I) entsteht. Wäre nun Z und R in der konkreten Reaktion identisch, so könnte keine von der Zwischenverbindung (III) verschiedene Verbindung entstehen.

In einer bevorzugten Ausführungsform des erfindungsgemäßen Verfahrens - und zwar sowohl der einstufigen als auch 2-stufigen Variante - werden die im Zuge der Reaktion gebildeten Verbindungen CH₃-O-CO-Z bzw. CH₃-CH₂-O-CO-Z und X-O-CO-Z kontinuierlich aus dem Reaktionsgemisch entfernt, insbesondere durch Destillation. Dieses kontinuierliche Abdestillieren hat den Vorteil, dass die Reaktion unter schonenden Bedingungen durchgeführt werden kann und hellfarbige Zielprodukte (I) liefert.

Das erfindungsgemäße Verfahren hat mehrere Vorteile: Man geht von preiswerten und gut zugänglichen Rohstoffen aus. Die im Zuge der Reaktion gebildeten Verbindungen CH₃-O-CO-Z bzw. CH₃-CH₂-O-CO-Z und X-O-CO-Z (Nebenprodukte) lassen sich einerseits gut abtrennen und andererseits als Wertprodukt nutzen (Methylacetat etwa lässt sich als Lösungsmittel verwenden). Im Übrigen lässt sich das erfindungsgemäße Verfahren unter schonenden Bedingungen durchführen.

Die Wahl der Umesterungs-Katalysatoren ist an sich nicht kritisch. Man kann an sich jeden Umesterungskatalysator einsetzen. Besonders geeignete Umesterungskatalysatoren sind Alkoholate wie Natriummethylat, Kaliummethylat oder Hydroxide wie Natriumhydroxid und Kaliumhydroxid sowie Alkalicarbonate. Die Katalysatoren werden vorzugsweise in einer Menge von 0,01- 5%, bevorzugt von 0,5-1% bezogen auf die gesamte Reaktionsmischung eingesetzt.
In der 2-stufigen Verfahrensweise setzt man vorzugsweise in beiden Schritten denselben Katalysator ein.

Die Reaktionstemperatur ist an sich nicht kritisch. Vorzugsweise wird sie zwischen 40 und 150°C durchgeführt. Als besonders bevorzugt hat sich der Bereich zwischen 60 und 110°C erwiesen.

Die Reaktanden werden vorzugsweise in stöchiometrischer Menge und insbesondere in einem leichten Überschuss des Dimethylcarbonat oder Diethylcarbonats miteinander umgesetzt.

In einer Ausführungsform der 2-stufigen Verfahrensweise wird in Schritt 1 die Verbindung (II) vorgelegt und Dimethylcarbonat oder Diethylcarbonat portionsweise oder kontinuierlich zudosiert, während die bei Einsatz von Dimethylcarbonat gebildete Verbindung CH₃-O-CO-Z bzw. die bei Einsatz von Diethylcarbonat gebildete Verbindung CH₃-CH₂-O-CO-Z kontinuierlich abdestilliert wird. In einer Ausführungsform der 2-stufigen Verfahrensweise wird in Schritt 2 die Verbindung (IV) portionsweise oder kontinuierlich zudosiert, während die gebildete Verbindung X-O-CO-Z kontinuierlich abdestilliert wird. Die beiden letztgenannten Ausführungsformen werden vorteilhafterweise kombiniert.

In einer Ausführungsform der 2-stufigen Verfahrensweise kann der Reaktand (IV) in Schritt 2 gewünschtenfalls im Überschuss eingesetzt werden um einen höheren Umsetzungsgrad zu erzielen. Nach Beendigung der Reaktion kann dieser Überschuss im Vakuum abdestilliert werden.

Beispiele für besonders geeignete Gruppen Z in den Verbindungen (II) sind Wasserstoff, Methyl, Ethyl, n-Propyl und i-Propyl. Methyl ist dabei ganz besonders bevorzugt - in diesem Falle handelt es sich bei (II) um Triacetin (= Triessigsäureester des Glycerins).

Beispiele für geeignete Gruppen X in den Verbindungen (IV) sind: Methyl, Ethyl, n-Propyl, i-Propyl, n-Butyl, i-Butyl, tert.-Butyl, n-Pentyl, n-Hexyl, n-Heptyl, n-Octyl, 2-Ethylhexyl. In einer bevorzugten Ausführungsform hat X in Formel (IV) die Bedeutung einer Methylgruppe.

Beispiele für geeignete Gruppen R in den Verbindungen (IV) sind: Wasserstoff, Methyl, Ethyl, n-Propyl, i-Propyl, n-Butyl, i-Butyl, tert.-Butyl, n-Pentyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Pentadecyl, n-Hexadecyl, n-Heptadecyl, n-Heptadecenyl, n-Octadecyl, n-Nonadecyl, n-Eicosyl, n-Heneicosyl, n-Docosyl sowie -C(CH₃)=CH₂ oder -CH=CH₂. Vorzugsweise leitet sich R in den Verbindungen (IV) von Fettsäuren mit 3 bis 24 C-Atomen ab.

In einer weiteren bevorzugten Ausführungsform hat in Formel (II) X die Bedeutung einer Methylgruppe und R die Bedeutung einer n-Heptylgruppe. In einer weiteren bevorzugten Ausführungsform hat in Formel (IV) X die Bedeutung einer Methylgruppe und R die Bedeutung einer Gruppe -C(CH₃)=CH₂ oder -CH=CH₂.

Die Verbindungen (IV) können einzeln oder im Gemisch untereinander eingesetzt werden.

Das erfindungsgemäße Verfahren kann gewünschtenfalls in Gegenwart eines Lösungsmittels durchgeführt werden. Vorzugsweise wird es jedoch in Abwesenheit eines Lösungsmittels durchgeführt.

Gewünschtenfalls kann das Zielprodukt (I) dem Fachmann vertrauten Reinigungsschritten unterworfen werden, um die Reinheit des Produktes zu erhöhen.

Ein weiterer Erfindungsgegenstand ist die Verwendung der Verbindungen (I) als Lösungsmittel oder als hochsiedende Arbeitsflüssigkeiten. Hierbei ist von Vorteil, dass das die Verbindungen (I) bei erhöhten Temperaturen durch Zersetzung CO₂ abgeben können (Flammschutzwirkung). Die Verbindungen (I) können außerdem für folgende Verwendungszwecke eingesetzt werden: Hydrophobe Emulgatoren, halogenfreie Schmiermittelzusätze, z. B. für Treibstoffe.

### Beispiele

### Beispiel 1:

### Herstellung von Glycerincarbonat-Methacrylat

Die Reaktion erfolgte im Eintopf-Verfahren, wobei eine Verbindung (II) (hier: Triacetin), Dimethylcarbonat und eine Verbindung (III) (hier: Methylmethacrylat) gleichzeitig miteinander umgesetzt wurden.

Ansatz: Es wurden folgende Mengen eingesetzt:

| | **mol. Gewicht** **(g/mol)** | **Menge** **(mol)** | **Menge** **(g)** |
|---|---|---|---|
| **Triacetin** | 218 | 0,5 | 109 |
| **Dimethylcarbonat (DMC)** | 90 | 0,5 | 45 |
| **NaOMe** | 54 | 0,02 | 1,08 (3,60g von einer 30%igen Lösung) |
| **Methyl Methacrylat (MMA)** | 100 | 0,5 | 50 |
| **HCl (2N)** | 36,5 | 0,02 | 10,2 |

Durchführung: Triacetin, DMC, MMA und NaOMe wurden gerührt und erhitzt um AcOMe abzudestillieren. Die Destillation wurde zwischen 75 und 110°C im Sumpf durchgeführt. Um die Ausbeute zu verbessern, wurde die Destillation unter Vakuum weitergeführt (von 500 bis 50 mbar und bis 100°C im Sumpf). Es wurde mit HCl neutralisiert und in der Wärme eine Phasentrennung durchgeführt. Die organische Phase wurde mit 28g Wasser ausgewaschen und getrocknet.
Es wurden 81,2g Produkt erhalten (= 87% Ausbeute). Reinheit (GC): 67% Glycerin-carbonat-Methacrylat. Das Produkt enthielt noch ca. 27% Glycerincarbonat-Acetat.

### Beispiel 2:

### Herstellung von Glycerincarbonat-Octanoat

Die Reaktion erfolgte im Eintopfverfahren in 2 Schritten: Zuerst wurde die Herstellung vom Glycerincarbonat-Acetat (Schritt 1) und dann die Herstellung des C-8 Derivates (Schritt 2) durchgeführt. Die Reaktion kann schematisch wie folgt veranschaulicht werden:

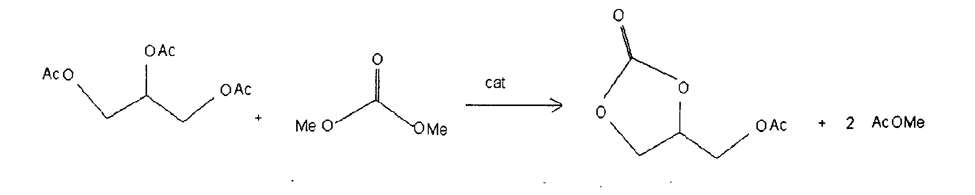

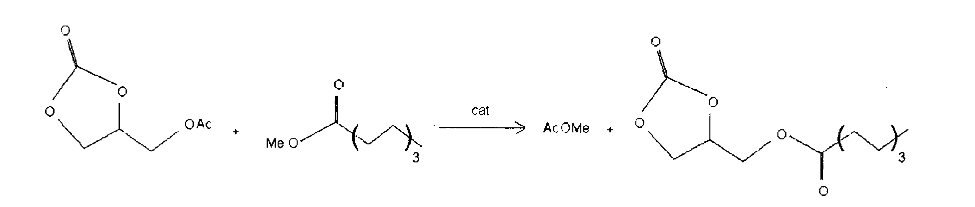

Ansatz: Es wurden folgende Mengen eingesetzt:

| | **mol. Gewicht** **(g/mol)** | **Menge** **(mol)** | **Menge** **(g)** | |
|---|---|---|---|---|
| **Triacetin** | 218 | 1 | 218 | |
| **Dimethylcarbonat** | 90 | 1,1 | 99 | 10% Überschuss |
| **NaOMe** | 54 | 0,059 | 3,19 (10,6g von einer 30%igen Lösung) | |
| **C-8 Methyl Ester** | 158,2 | 1,1 | 175 | 10% Überschuss |
| **HCl (2N)** | 36,5 | 0,059 | 30 | |
| **NaHCO₃** | 84 | 0,059 | 5 (50g von einer 10%igen Lösung) | |
| **Gesättigte NaCl Lösung** | - | - | 50,5+50,5 | |

### Durchführung:

Erste Stufe: DMC, Triacetin und NaOMe wurden unter Stickstoff-Atmosphäre erhitzt um AcOMe abzudestillieren. Die Destillation wurde zwischen 62 und 100°C im Sumpf durchgeführt. Destillatmenge: 159,5g (theoretische Menge: 164,4g).
Zweite Stufe: C-8 Methylester (Methylester von n-Octansäure) wurde hinzugefügt. AcOMe wurde unter Wasserstrahlvakuum zwischen 40 und 100°C im Sumpf abdestilliert.
Es wurde mit HCl neutralisiert. Die saure Mischung wurde mit einer 10%ige NaHCO₃ Lösung rückneutralisiert. Eine erste Phasentrennung wurde in der Wärme durchgeführt. 50,5g einer gesättigten NaCl Lösung wurden hinzugefügt, geschüttelt und eine weitere Phasentrennung durchgeführt.
Gesamtmenge der abgetrennten wässeriger Phase: 142,6g (gesamte Menge von Wasser hinzugefügt: 130,5g). Die organische Phase wurde ein weiteres Mal mit 50,5g gesättigter NaCl Lösung ausgewaschen und das Produkt im Wasserstrahlvakuum zwischen 40 und 80°C im Sumpf getrocknet.
Es wurden 164g (= 67% Ausbeute) Glycerincarbonat-Octanoat mit einer gaschromatographisch bestimmten Reinheit von 90% erhalten.

## Patentansprüche

1. Verfahren zur Herstellung von Glycerincarbonat-Estern der Formel (I) 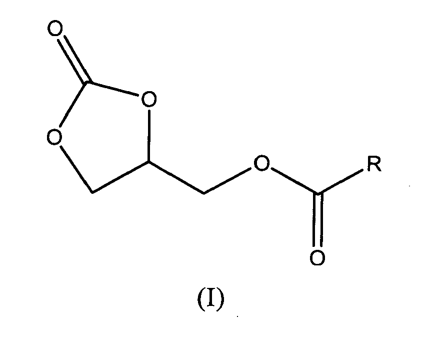 worin R Wasserstoff oder einen Alkyl- oder Alkenylrest mit 1 bis 23 C-Atomen bedeutet, der geradkettig oder verzweigt sein kann, wobei man in Gegenwart eines Umesterungs-Katalysators eine Verbindung der Formel (II) 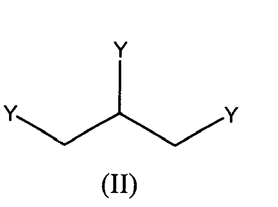 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat und einem Alkylester der Formel (IV) 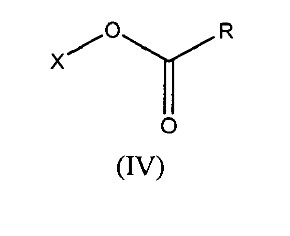 worin R die oben genannte Bedeutung hat und X einen Alkyl- oder Alkenylrest mit 1 bis 8 C-Atomen bedeutet, der linear oder verzweigt sein kann, umestert, mit der Maßgabe, dass die Gruppe R der Verbindung (IV) verschieden von der Gruppe Z der Verbindung (II) ist.

2. Verfahren zur Herstellung von Glycerincarbonat-Estern der Formel (I) 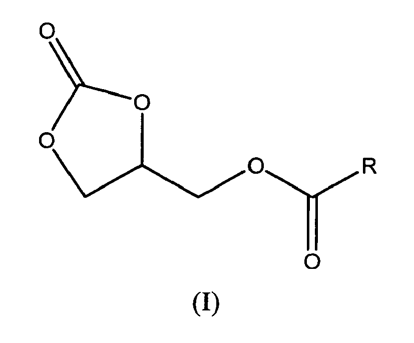 worin R Wasserstoff oder einen Alkyl- oder Alkenylrest mit 1 bis 23 C-Atomen bedeutet, der geradkettig oder verzweigt sein kann, wobei man in einem ersten Schritt in Gegenwart eines Umesterungs-Katalysators eine Verbindung der Formel (II) 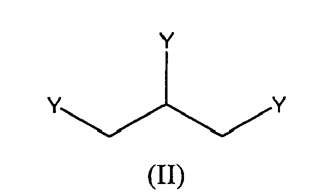 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat zu einer Verbindung der Formel (III) 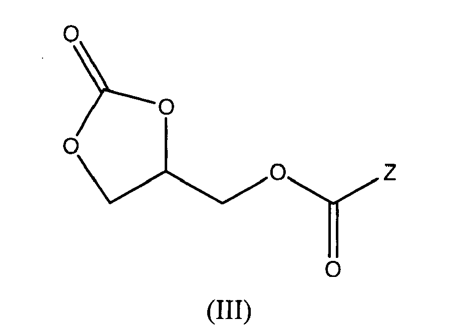 worin Z die oben genannte Bedeutung hat, umsetzt und die Verbindung (III) in einem zweiten Schritt in Gegenwart eines Umesterungs-Katalysators mit einem Alkylester der Formel (IV) 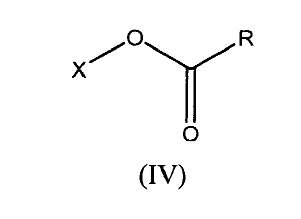 worin R die oben genannte Bedeutung hat und X einen Alkyl- oder Alkenylrest mit 1 bis 8 C-Atomen bedeutet, der linear oder verzweigt sein kann, umestert, mit der Maßgabe, dass die Gruppe R der Verbindung (IV) verschieden von der Gruppe Z der Verbindung (II) ist.

3. Verwendung der Verbindungen (I) als Lösungsmittel, Arbeitsflüssigkeiten mit Flammschutzwirkung oder Schmiermittelzusatz für Treibstoffe.
